# EUROPEAN PATENT APPLICATION

(11) **EP 4 813 424 A2**
(43) Date of publication of application: **30.09.2026**
(21) Application number: 26198816.6
(22) Date of filing: 23.11.2022
(51) Int. Cl.: A61M 5/315

(54) **A SUBASSEMBLY OF A MEDICAMENT DELIVERY DEVICE**

(30) Priority: 07.12.2021 EP 21212721
(62) Document number(s) of the earlier application(s) in accordance with Art. 76 EPC: 22821947.3 / 4 444 387
(71) Applicant: SHL Medical AG, 6302 Zug (CH)
(72) Inventor: CHIOU, Meng-Jhen, 338 Taoyuan City (TW); CHAO, Pei Yu, 330063 Taoyuan City (TW)

(57) **Abstract**

The present disclosure generally relates to medicament delivery devices such as autoinjectors, and particularly concerns a subassembly adapted to prevent injuries to a user caused by a medicament delivery member at a proximal end of the medicament delivery device.

## Description

### TECHNICAL FIELD

The present disclosure generally relates to medicament delivery devices such as autoinjectors, and particularly concerns a subassembly adapted to prevent injuries to a user caused by a medicament delivery member at a proximal end of the medicament delivery device.

### BACKGROUND

A number of medical conditions require injections. These days, a number of different injection devices exist, including various types of pen injectors, autoinjectors and on-body devices. Although many of these devices have enabled major improvements in the management of a number of medical conditions, various limitations do still exist in the current technology. Not least amongst these are the difficulties faced by patients that require frequent injections and by patients that need to inject particularly viscous drugs. In considering these problems, the applicant has appreciated that various developments could be made to help improve the medicament delivery devices on the market today, for example concerning preventing injuries to a user caused by a needle of the medicament delivery device, which are set out in more detail below.

### SUMMARY

An object of the present disclosure is to provide a subassembly for a medicament delivery device which solves, or at least mitigates problems of the prior art.

According to a first aspect of the present disclosure, there is provided a subassembly of a medicament delivery device for expelling medicament from a medicament container, the subassembly comprising: a housing having a proximal end and a distal end, the housing comprising at least one flexible portion adapted to flex in a radial direction; a cover structure configured to surround a medicament delivery member at a proximal end of the medicament delivery device, the cover structure is movable inside the housing in an axial direction of the housing between a retracted position in which the medicament delivery member is exposed at the proximal end of the cover structure, and an extended position in which the medicament delivery member is covered by the cover structure, the cover structure comprising a flexible member at a distal end of the cover structure, the flexible member is adapted to flex in a radial direction and comprises a guide protrusion protruding radially inwards and a locking protrusion protruding radially outwards; a rotator arranged radially inside and coaxially with the cover structure and comprising a guiding track configured to receive the guide protrusion, the guiding track being adapted so that when the cover structure is moved towards the retracted position, the guide protrusion interacts with and slides in the guiding track from an initial position corresponding to the extended position of the cover structure to an intermediate position corresponding to the retracted position of the cover structure while causing the rotator to rotate about a longitudinal axis of the medicament delivery device, wherein once in the intermediate position, the guide protrusion reaches a locking position in a locking portion of the rotator when the cover structure is moved from the retracted position to the extended position, in the locking portion, the rotator comprises a ramp surface adapted to interact with the guide protrusion to push the flexible member radially outwards so that the locking protrusion encounters the at least one flexible portion of the housing so that the cover structure is prevented to move towards the retracted position.

Embodiments of the present disclosure advantageously provide for a lockout function for the cover structure so that once a user has used the medicament delivery device, thus having pushed the cover structure to the retracted position, and subsequently released it to the extended position, the guide protrusion has reached a locking portion of the rotator. In the locking portion, a ramp surface causes the flexible member of the cover structure to be pushed radially outwards to be locked in place by interaction with a flexible portion of the housing. Once in this locked position, the cover structure is prevented from moving to the extended position. In other words, when the cover structure is for the first time moved to the retracted position, the guide protrusion travels along the guiding track and causes a rotation of the rotator so that when the cover structure is moved back towards the extended position, the guide protrusion travels along a different path to reach the locking portion instead of the initial position. Once in the locking portion the cover structure cannot easily be moved back to the retracted position due to the interaction between the flexible member of the cover structure and the flexible portion of the housing. In this way, after use, the cover structure is maintained in the extended position such that a user is prevented from sharp injury caused by a medicament delivery member, such as a needle.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the components thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", "circumferentially" refer to a circumference or a circumferential direction relative to an axis, typically a central axis extending in the direction of the longest extension of the device and/or component. Similarly, "radial" or "radially" refer to a direction extending radially relative to the axis, and "rotation", "rotational" and "rotationally" refer to rotation relative to the axis.

The same direction terminology has been used to describe other components such as the pad - for example, the proximal end of the pad is the part of the pad closest to the dose delivery site (injection site), and the distal end of the pad is the part of the pad furthest from the dose delivery site. In the Figures, the longitudinal direction is the direction of axis 102, with the corresponding circumferential direction 31 and radial direction 32 relative to the axis 102 also shown.

When the wording 'at the injection site' or 'at the dose delivery site' is used in this application, it generally refers to the point where the medicament delivery device (e.g., a needle) enters the patient, along with the surrounding area, for example the area where the pad is attached.

According to one embodiment, in an initial extended position of the cover structure with the guide protrusion in the initial position, the locking protrusion of the cover structure and the at least one flexible portion of the housing are not in contact with each other. In other words, before the cover structure has been moved to the retracted position, the rotator is in its original orientation, and the guide protrusion of the cover structure is not in the locking portion and thereby not on the ramp surface of the locking portion. When the guide protrusion is not on the ramp surface of the locking portion, the locking protrusion is not pushed radially outwards and does not reach the flexible portion of the housing. Thus, there is still a gap between the locking protrusion of the cover structure and the flexible member of the housing thereby allowing free motion between them before lockout.

According to one embodiment, the cover structure may comprise at least one guiding rib configured to slide in corresponding guiding slots of the housing when the cover structure moves in the axial direction. The guiding slots are preferably parallel with the longitudinal axis of the medicament delivery device. Since a gap may exist between the housing and the cover structure, the guiding ribs and guiding slots ensure guidance of the cover structure in the housing.

According to one embodiment, the cover structure may comprise two guiding ribs for each flexible member, where two guiding ribs are arranged with a respective flexible member between them. Additional guiding ribs provide for improve guiding of the cover structure. Pairs of guiding ribs are preferably parallel.

According to one embodiment, the at least one flexible portion of the housing may comprise a respective protrusion facing radially inwards towards the cover structure, the protrusion of the flexible portion comprising a ramp surface arranged to receive the locking protrusion of the cover structure when the locking protrusion moves towards the locking portion. The ramp surface of the protrusion of the housing is arranged on a distal end of the protrusion, so that when the locking protrusion approaches the protrusion of the housing when the cover structure moves towards the extended position, the ramp surface of the housing protrusion receives the locking protrusion of the cover structure. The ramp surface provides for facilitating pushing the protrusion of the flexible portion of the housing to flex outwards when interacting with the locking protrusion of the cover structure.

Thus, the protrusion of the flexible member of the cover structure causes the flexible portion of the housing to flex radially outwards when the locking protrusion of the flexible member slides on the ramp surface.

Further, the flexible portion of the housing is adapted to flex back to an original position once the locking protrusion of the flexible member has moved to a position proximal in relation to the flexible portion. Thus, once the locking protrusion of the cover structure has moved past the flexible portion of the housing, including the protrusion thereof, to a location proximal of the protrusion, the flexible portion flexes back and locks the locking protrusion in place and therefore also the cover structure is locked.

According to one embodiment, the protrusion of the flexible portion of the housing comprises a proximal surface configured to interact with the locking protrusion of the flexible member of the cover structure so that the cover structure is locked in the extended position. The proximal surface is opposite the ramp surface of the protrusion of the flexible portion of the housing.

According to one embodiment, the guide protrusion of the cover structure may comprise a ramp surface on a proximal side of the guide protrusion configured to interact with the ramp surface of the rotator. The ramp surface of the guide protrusion facilitates the motion of the guide protrusion up the ramp surface of the rotator.

According to one embodiment, the at least one flexible member of the cover structure comprises a free end and a fixed end, wherein the free end is adapted to flex in the radial direction.

According to one embodiment, the free end may be arranged on the distal-most end of the cover structure.

According to one embodiment, the flexible member of the cover structure may be made from a cut-through in a distal end of the cover structure.

According to one embodiment, the flexible portion of the housing may be made from a cut-through in the housing.

Hereby, by means of a cut-through, a relatively simple way of providing the cover structure with the flexible member and the housing with the flexible portion is provided.

According to one embodiment, the cover structure may comprise two flexible members symmetrically arranged on opposite sides of the rotator. This provides more stability and reliability of the lockout of the cover structure.

According to one embodiment, the locking portion may be a locking slot comprising side walls to guide the guide protrusion.

There is further provided a medicament delivery device comprising the subassembly of any of the herein disclosed embodiments.

Generally, all terms used in the claims are to be interpreted according to their ordinary meaning in the technical field, unless explicitly defined otherwise herein. All references to "a/an/the member, apparatus, component, means, etc." are to be interpreted openly as referring to at least one instance of the member, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Figs. 1A-B are perspective views of an autoinjector according to embodiments of the present disclosure;
Fig. 2 illustrates an exploded view of the subassembly in Figs. 1A-B;
Fig. 3 illustrates the rotator and the distal end of the cover structure according to embodiments of the present disclosure;
Fig. 4 illustrates the rotator and the distal end of the cover structure with the guiding protrusion received in the guiding track according to embodiments of the present disclosure;
Fig. 5 illustrates the rotator and the distal end of the cover structure with the guiding protrusion in the guiding track according to embodiments of the present disclosure;
Fig. 6 illustrates the rotator and the distal end of the cover structure with the guiding protrusion in an intermediate position in the guiding track according to embodiments of the present disclosure;
Fig. 7 illustrates the rotator and the distal end of the cover structure with the flexible member of the cover structure pushed radially outwards by a ramp surface of the rotator according to embodiments of the present disclosure;
Fig. 8 is a cross-section of the rotator and the distal end of the cover structure with the flexible member of the cover structure pushed radially outwards by a ramp surface of the rotator to encounter the flexible member of the housing according to embodiments of the present disclosure;
Fig. 9 is a cross-section of the rotator and the distal end of the cover structure with the flexible member of the cover structure pushed radially outwards by a ramp surface of the rotator to encounter the flexible member of the housing according to embodiments of the present disclosure;
Fig 10 illustrates the flexible member of the cover structure and the flexible portion of the housing according to embodiments of the present disclosure;
Fig. 11 illustrates an exploded view of a subassembly;
Fig. 12 illustrates the rotator and distal part of the cover structure of the subassembly shown in fig. 11;
Fig. 13 illustrates the rotator and distal part of the cover structure of the subassembly shown in fig. 11 when the cover structure is locked in a locked position; and
Fig. 14 illustrates the rear cap of the subassembly shown in fig. 11.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like members throughout the description.

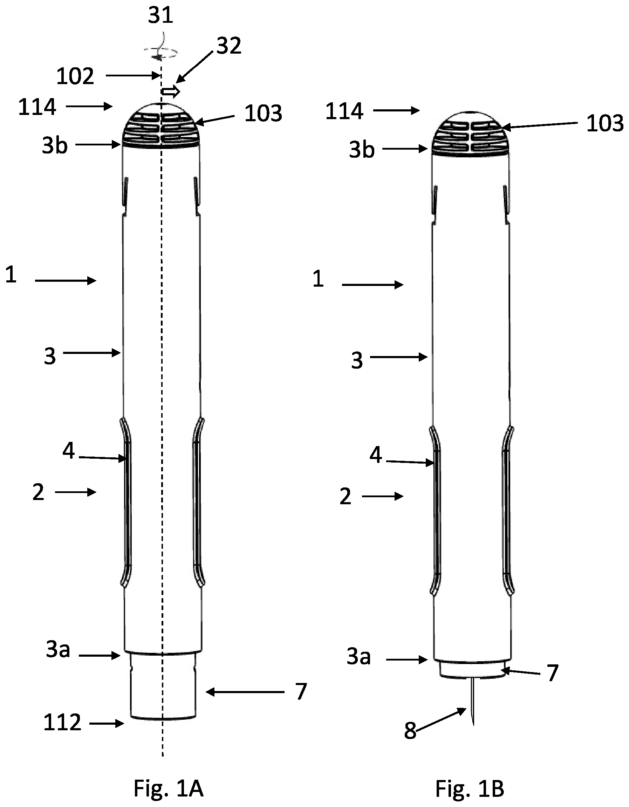

Figs 1A and 1B show an example of a medicament delivery device 1 such as an autoinjector according to embodiments of the present disclosure. The medicament delivery device 1 is configured to expel medicament from a medicament container via a medicament delivery member 8 to a user at a dose delivery site. The medicament delivery device 1 extends from a proximal end 112 to a distal end 114 relative to the axis 102.

The medicament delivery device 1 comprises a housing 3 with a window 4 and a flexible portion 5 that is adapted to flex in transversal direction 32 of the medicament delivery device. The housing 3 has a proximal end 3a and a distal end 3b. A rear cap 103 covers the distal end 3b of the housing 3.

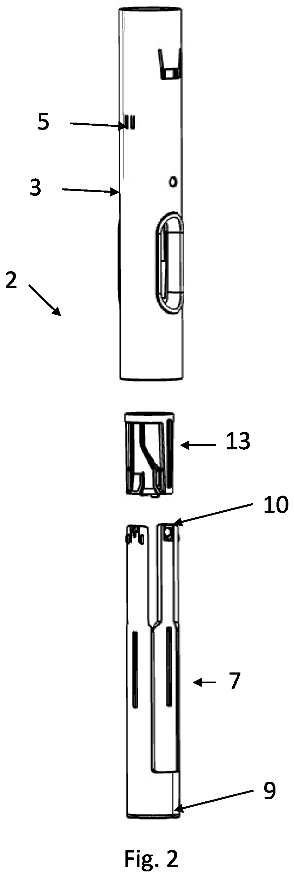

The medicament delivery device 1 comprises a cover structure 7. The cover structure 7 is arranged in the housing 3 and extends proximally from the proximal end 3a. The housing 3 and the cover structure 7 is part of a subassembly 2 together with a rotator better seen in e.g., fig. 2.

The cover structure 7 is configured to be moved linearly relative to the housing 3 from an extended position shown in Fig. 1A to a retracted position shown in fig. 1B in which the cover structure 7 is received further in the housing 3 and in which a medicament delivery member such as a needle 8 is exposed at the proximal end 3a of the housing 3.

The cover structure 7 is biased in the proximal direction towards the extended position shown in fig. 1A.

The medicament delivery device 1 comprises the subassembly 2 which will now be described in more detail with reference to subsequent drawings.

Fig. 2 is an exploded view of the subassembly 2 according to embodiments of the present disclosure. The subassembly comprises the housing 3 the cover structure 7, and the rotator 13 which here have been axially displaced in the drawing to better illustrate different parts of the subassembly 2.

As described above, the cover structure 7 is configured to surround a medicament delivery member at a proximal end 112 of the medicament delivery device. The cover structure 7 is movable inside the housing 3 in an axial direction of the housing 3 between a retracted position in which the medicament delivery member is exposed at the proximal end 9 of the cover structure 7, and an extended position in which the medicament delivery member is covered by the cover structure 7.

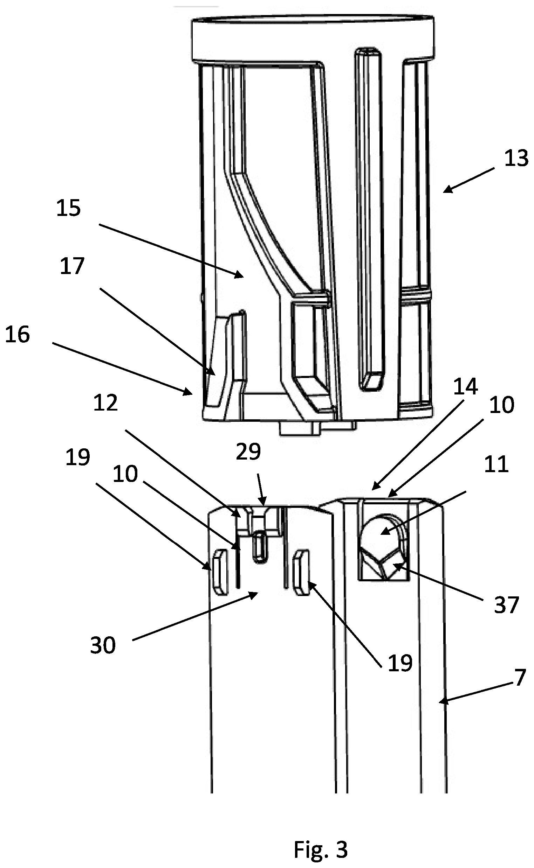

Turning to fig. 3, the cover structure 7 comprises a flexible member 10 at a distal end 14 of the cover structure 7. The flexible member 10 is adapted to flex in a radial direction 32 and comprises a guide protrusion 11, protruding radially inwards and a locking protrusion 12 protruding radially outwards.

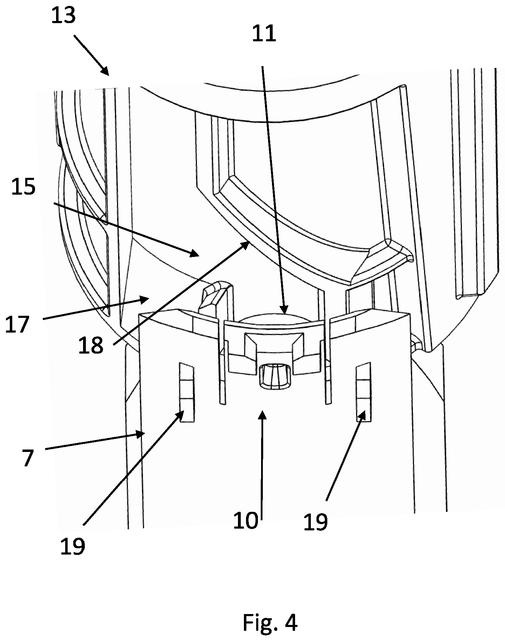

Further, the rotator 13 is arranged radially inside and coaxially with the cover structure 7 and comprises a guiding track 15 configured to receive the guide protrusion 11. Fig. 4 illustrates the flexible member 10 and the guide protrusion 11 in a position where the cover structure 7 has moved partly towards the retracted position, and where the guide protrusion 11 is received in the guiding track 15.

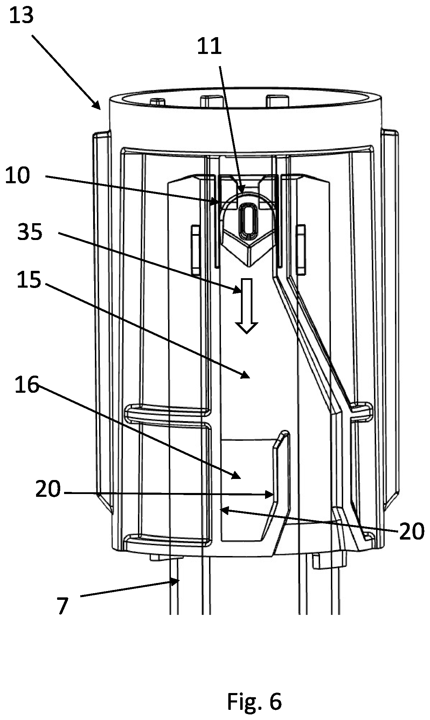

The guiding track 15 being adapted so that when the cover structure 7 is moved towards the retracted position, the guide protrusion 11 interacts with and slides in the guiding track 15 from an initial position corresponding to the extended position of the cover structure 7, shown in fig. 4, to an intermediate position, shown in fig. 6, corresponding to the retracted position of the cover structure while causing the rotator to rotate about a longitudinal axis 102 of the medicament delivery device 1.

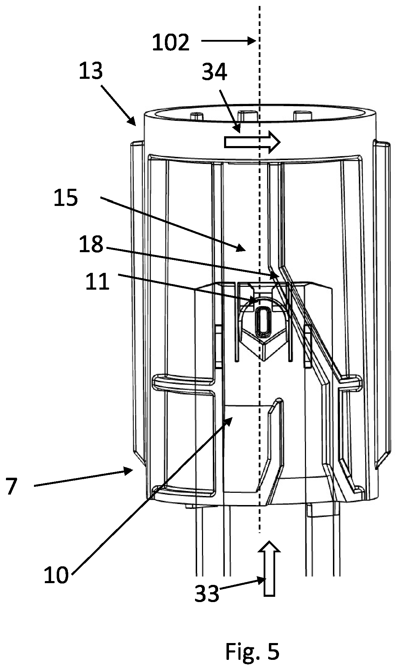

More precisely, the guide protrusion 11 is first in an initial position shown in fig. 4. Here, the guide member 11 is at the initial proximal part of the guiding track 15. Turning to fig. 5, as the cover structure 7, here shown transparent to better display the guide protrusion 11 in the track 15, moves in the distal direction 33 towards the retracted position, the guide protrusion 11 travels in the guiding track 15 and interacts with the side wall 18 and thereby causes the rotator 13 to rotate about the axis 102 in the direction indicated by arrow 34. The inclination of the sidewall 18 with respect to the direction 33 of the cover structure movement enables for a transversal force to be applied by the guide member 11 on the rotator 13 so that the rotator 13 rotates.

As shown in fig. 6, the guide protrusion 11 reaches the intermediate position at the distal end of the guiding track 15 when the cover structure 7 is pushed further into the housing 3. In this position, the cover structure is in the retracted position.

When the cover structure 7 subsequently moves from the retracted position towards the extended position, the guide protrusion 11 will travel in the guiding track 15 in a direction 35 towards a locking portion 16 of the rotator 13. Thus, once in the intermediate position, the guide protrusion 11 reaches a locking position in a locking portion 16 of the rotator 13 when the cover structure 7 is moved from the retracted position to the extended position.

The guide protrusion 11 travels freely in a straight, longitudinal path towards the locking portion 16 and no rotation of the rotator 13 is caused by that motion of the cover structure 7 and the guide protrusion 11. Thus, the guide protrusion 11 and the guiding track 15 are configured so that once the guide protrusion 11 reaches the intermediate position, it will during the subsequent motion of the cover structure 7 towards the extended position, reach the locking portion 16.

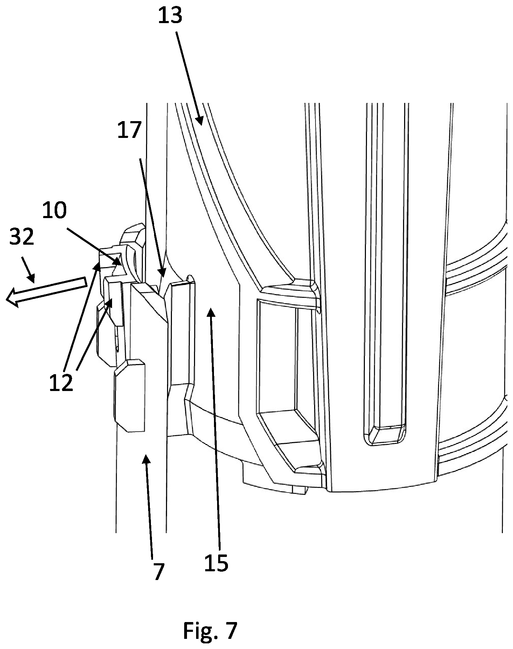

Turning again to fig. 3 in conjunction with fig. 7, in the locking portion 16, the rotator 13 comprises a ramp surface 17 adapted to interact with the guide protrusion 11 to push the flexible member 10 radially outwards 32 so that the locking protrusion 12 encounters the at least one flexible portion 5 of the housing 3 so that the cover structure 7 is prevented to move to the retracted position.

As best seen in fig. 6, the locking portion 16 is made as a locking slot comprising side walls 20 to guide the guide protrusion 11. Further, the side walls 20 prevent cover structure 7 from rotating out from the locked position since the guide protrusion 11 is held in place by the side walls 20. The side walls 20 extend in a direction substantially parallel with the longitudinal direction 102 of the medicament delivery device 1.

As is better seen in fig 7, due to that the guide protrusion 11 travels proximally up the ramp surface 17, the flexible member 10 which includes the guide protrusion 11 is resiliently flexed radially outwards 32. The ramp surface 17 has its bottom in a distal end of the ramp surface 17 and reaches further radially outwards on the more proximally portions of the ramp surface 17, thereby resulting a larger the diameter of the rotator 13 on more proximal parts of the ramp surface 17.

As the flexible member 10 is pushed radially outwards, so are the locking protrusions 12 protruding radially outwards towards an inner side of the housing 3.

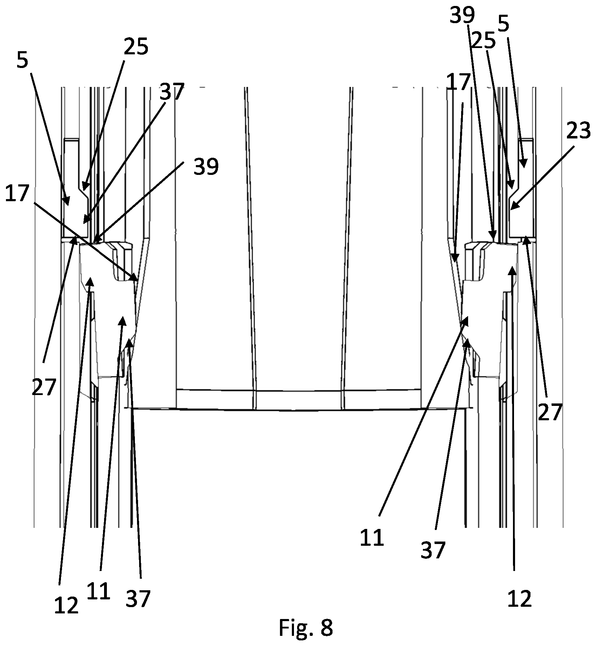

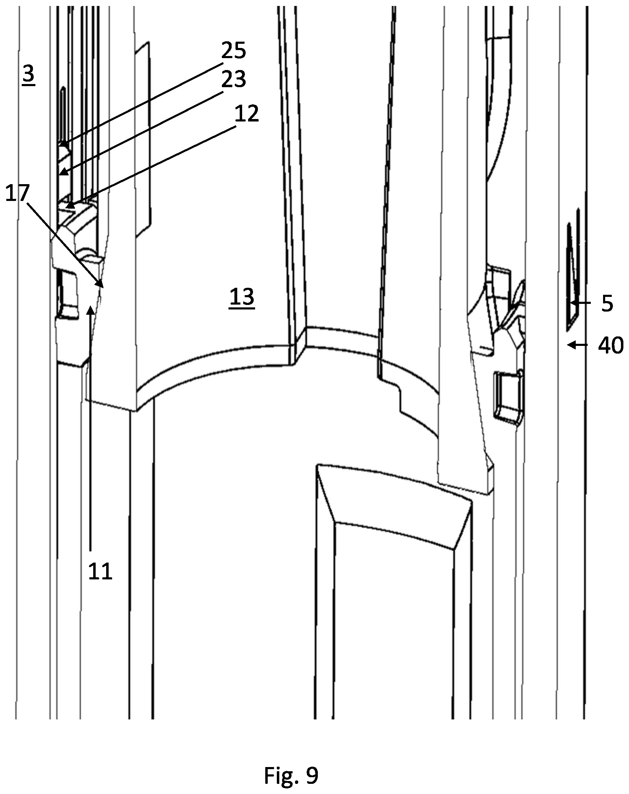

Fig. 8 and 9 are cross-sections of the subassembly 2 with the guide protrusion 11 in the locking portion 16, where the ramp surface 17 has forced the flexible member 10 outwards, as also illustrated in fig. 7. The locking protrusions 12 have reached radially outside the protrusions 23 of the flexible member of the housing 5. In other words, as the cover structure 7 was moved to the extended position, the locking protrusions 12 have encountered the flexible portion, more precisely, the protrusions 23 of the flexible portion 5.

It should be noted that in an initial extended position of the cover structure 7 shown in fig. 4, with the guide protrusion in the initial position, the locking protrusion 12 of the cover structure 7 and the at least one flexible portion 5 of the housing are not in contact with each other.

The respective protrusion 23 of the flexible portion 5 faces radially inwards towards the cover structure 7 and the rotator 13. Further, the protrusion 23 of the flexible portion 5 comprises a ramp surface 25 arranged to receive the locking protrusion 12 of the cover structure 7 when the locking protrusion moves towards the locking portion 16. When the cover structure moves towards the extended position, the locking protrusion 12 slide on the ramp surface 25 to thereby more easily move past the protrusion 23 to a location proximal to the protrusion 23 as shown in fig. 8. When the locking protrusion 12 slides on the ramp surface 25, it causes the flexible portion 5 of the housing 5 to flex radially outwards.

The flexible portion 5 of the housing is resilient, whereby the flexible portion 5 flexes back to an original position, shown in fig. 8, once the locking protrusion 12 of the flexible member 10 has moved to a position proximal in relation to the flexible portion 5. In this original position, the flexible portion 5 may be flush with an outer surface 40 of the housing 3, as better seen in fig. 9.

Further, again with reference to fig 8, the protrusion 23 comprises a proximal surface 27 being configured to interact with the locking protrusion 12 of the flexible member 10 of the cover structure 7 so that the cover structure 7 is locked in the extended position. If the cover structure 7 is attempted to be moved in the distal direction towards the retracted position, a distal surface 39 of the locking protrusion 12 abuts with the proximal surface 27 of the protrusion 23 of the flexible member 5 and prevents further distal motion of the cover structure 7.

Further, in order to improve the sliding motion of the guide protrusion 11 of the cover structure 7 on the ramp surface 17, the guide protrusion 11 comprises a ramp surface 37 on a proximal side of the guide protrusion configured to interact with the ramp surface 17 of the rotator.

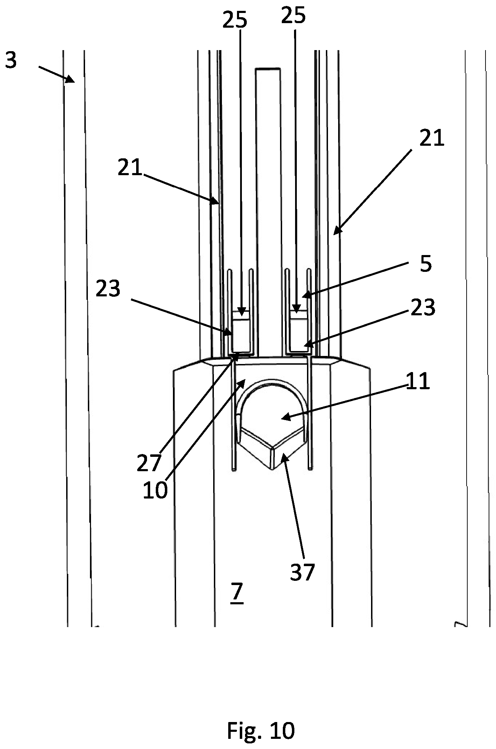

With reference to fig. 3, the at least one flexible member 10 of the cover structure comprises a free end 29 and a fixed end 30. The free end 29 is adapted to flex in the radial direction 32 of the medicament delivery device 1. The free end 29 is arranged on the distal-most end 14 of the cover structure 7. The flexible member 10 of the cover structure 7 can for example be made from a cut-through in a distal end 14 of the cover structure 7. In a similar way, the flexible portion 5 of the housing 3 can be made from a cut-through in the housing 3, better seen in fig. 10.

Further, the cover structure 7 preferably comprises two flexible members 10 symmetrically arranged on opposite sides of the rotator 13.

Turning to fig. 10 and fig. 3, the cover structure 7 comprises at least one guiding rib 19. The guiding rib 19 protrudes from the flexible member 10 in a direction opposite the protrusion direction of the guide protrusion 11. Thus, the guiding ribs 19 protrude towards the housing 3. Further, the housing 3 comprises corresponding guiding slots 21. When the cover structure moves in the housing in the axial direction, the guiding ribs 19, which fit in the slots 21, are configured to slide in the corresponding guiding slots 21 of the housing 3.

Preferably, each flexible member 10 is flanked by with two guiding ribs 19. Two guiding ribs 19 are thus arranged with a respective flexible member 10 between them.

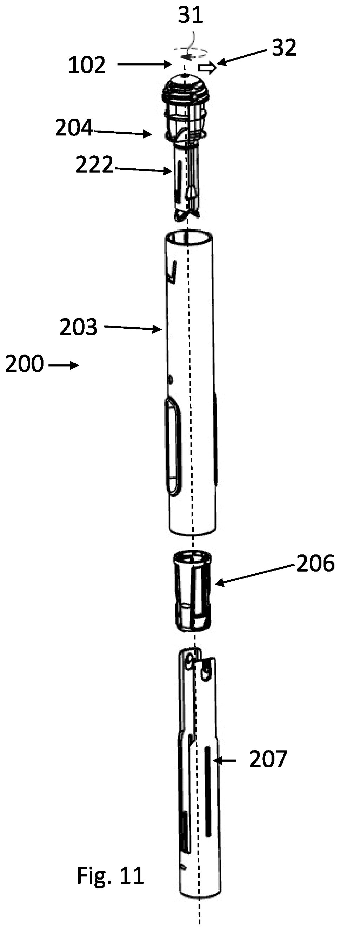

Fig. 11 is an exploded view of another subassembly 200 comprising a housing 203, a rear cap 204, a rotator 206, and a cover structure 207. The cover structure 207 is configured to surround a medicament delivery member at a proximal end of the medicament delivery device comprising the cover structure 207. The cover structure 207 is movable inside the housing 203 in an axial direction of the housing 203 between a retracted position in which the medicament delivery member is exposed at the proximal end of the cover structure 207, and an extended position in which the medicament delivery member is covered by the cover structure 207.

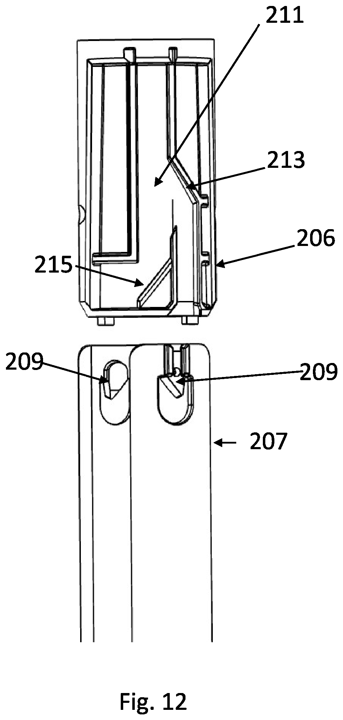

Reference should now be made to fig. 12 illustrating the rotator 206 and a distal part of the cover structure 207. The cover structure 207 comprises a guide pin 209 protruding towards the rotator 206. The rotator 206 comprises a track 211 in which the pin 209 slides when the cover structure 207 is moved from the extended position to the retracted position. When the pin 209 interacts with the wall 213 of the track 211, the rotator 206 rotates about its longitudinal axis 102. When the cover structure 207 is subsequently moved towards the extended position, the pin 209 interacts with the ramp 215, provided in the form of an inclined wall, to thereby cause further rotation of the rotator 206.

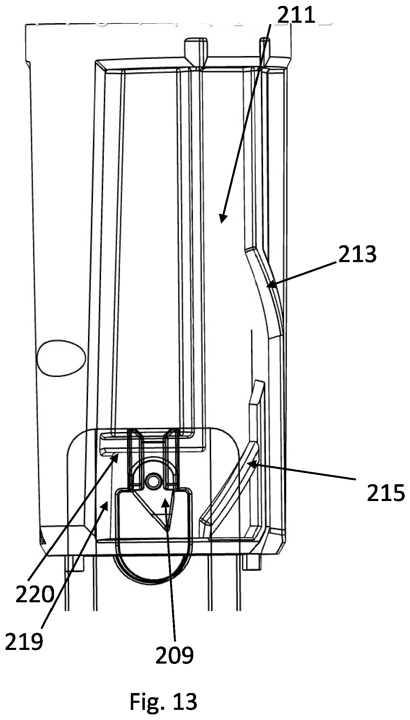

Turning to fig. 13, once in the extended position after use, the pin 209 is in the locking region 219 including a locking rib 220 or wall which receives the pin 209 if the cover structure 207 is again moved towards the retracted position. Thus, the rotators rib 220 locks the cover structure 207 in the extended position and thereby prevents injury caused by mistakenly pushing the cover structure 207 to the retracted position.

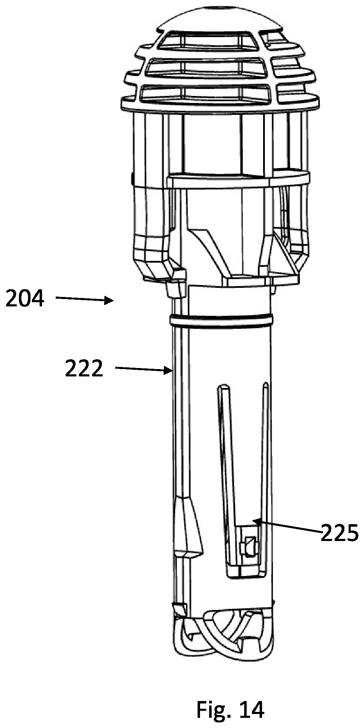

A closer view of the rear cap 204 is shown in fig. 14. The extended body 222 of the rear cap 204 is arranged inside the rotator 206, as illustrated in the exploded view o fig. 11. To facilitate for the rotation of the rotator 206 around the rear cap 204, the extended body that reaches inside the rotator 206 is provided with at least one chamfer 225.

An autoinjector may generally include various other components. For example, a sensor unit which may recognize injection events, such as the autoinjector inserted into an attachment portion of e.g., a pad, injection started, and injection ends, a memory unit which is configured to store the recorded data during the injection, a connectivity unit configured to transmit the stored data to a smart device or the network directly, a processing unit configured to control the entire system and processes the data before transmitting it, and/or user interface units that are configured to provide feedback to the patient, such as status LEDs, haptic, and/or audio feedback.

When the auto-injector is placed into the attachment portion, the sensors inside of the support pad are configured to recognize the event and give feedback to the patient via haptic/visual or audio elements.

When the injection finishes, the sensors are configured to recognize the event and give feedback to the patient again. Further, the collected data is stored in the memory unit and may be transmitted to the smart device/network via the connectivity unit after the injection event finishes.

The sensor can be one of or the combination of the following: a mechanical switch, a Hall-effect sensor, an accelerometer.

The mechanical switch, hall-effect sensor, or accelerometer can be used for detection of the insertion of the auto-injector into an injection port.

The accelerometer can be used for detecting injection events.

Possible wireless communication methods include Bluetooth and Cellular Networks.

Bluetooth connectivity requires a smart device to transmit the stored data to the network and it requires a pairing action between the support pad and the smart device before being able to use the supporting pad. But it's a cheaper alternative and it requires less space on PCB.

The cellular network does not require any pairing process, it can be used as a plug-n-play device, no prior setup is needed, but it's more expensive and it requires more space on PCB.

Depending on the requirements of the product any of those two technologies can be used.

Such processing units may comprise a logic circuit or control unit including a microprocessor, microcontroller, programmable digital signal processor or another programmable device. The processing circuitry may also, or instead, each include an application specific integrated circuit, a programmable gate array or programmable array logic, a programmable logic device, or a digital signal processor. Where the processing circuitry includes a programmable device such as the microprocessor, microcontroller or programmable digital signal processor mentioned above, the processor may further include computer executable code that controls operation of the programmable device.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A subassembly (200) of a medicament delivery device for expelling medicament from a medicament container, the subassembly comprising:
a housing (203) having a proximal end and a distal end;
a cover structure (207) configured to surround a medicament delivery member at a proximal end of the medicament delivery device, the cover structure (207) is movable inside the housing (203) in an axial direction of the housing (203 between a retracted position in which the medicament delivery member is exposed at the proximal end of the cover structure (207), and an extended position in which the medicament delivery member is covered by the cover structure (207),
the cover structure (207) comprising a guide protrusion (209) protruding radially inwards;
a rotator (206) arranged radially inside and coaxially with the cover structure (207) and comprising a guiding track (211) configured to receive the guide protrusion (209), the guiding track (211) being adapted so that when the cover structure (207) is moved towards the retracted position, the guide protrusion (209) interacts with an inclined wall (213) of the track (211) and slides in the guiding track (211) from an initial position corresponding to the extended position of the cover structure (207) to an intermediate position corresponding to the retracted position of the cover structure (207) while causing the rotator (206) to rotate about a longitudinal axis (102) of the medicament delivery device, wherein the guide protrusion (209) reaches a locking position in a locking portion (219) of the rotator (206) when the cover structure (207) is moved from the retracted position to the extended position, wherein,
in the locking portion (219), the rotator (206) comprises a ramp surface (215) adapted to interact with the guide protrusion (209) to further rotate the rotator 206 about the longitudinal axis (102) so that the cover structure (207) is prevented to move towards the retracted position by a locking rib (220).

2. The subassembly (200) according to claim 1, wherein the locking rib (220) is arranged distally of the guide protrusion (209) when the guide protrusion (209) is in the locking position.

3. The subassembly (200) according to claim 1 or 2, wherein the locking rib (220) extends horizontally.

4. The subassembly (200) according to any one of the preceding claims, wherein the locking rib (220) is a wall of the rotator (206).

5. The subassembly (200) according to any one of the preceding claims, wherein the guide protrusion (209) abuts the locking rib (220) when the guide protrusion (209) is in the locking portion (219) and the cover structure (207) is urged towards the retracted position.

6. The subassembly (200) according to any one of the preceding claims, wherein the rotator (206) rotates in the same rotational direction when the cover structure (207) is moved from the initial position to the intermediate position and from the intermediate position to the locking position.

7. The subassembly (200) according to any one of the preceding claims, further comprising a rear cap (204) configured to cover the distal end of the housing (203), the rear cap (204) comprising and extended body (222) arranged coaxially inside the rotator (206).

8. The subassembly (200) according to claim 7, wherein the extended bosy (222) comprises at least one chamfer (225) configured to facilitate the rotation of the rotator (206).

9. The subassembly (200) according to any one of the preceding claims, wherein the cover structure comprises two guide protrusions (209) symmetrically arranged on opposite sides of the rotator (206).

10. A medicament delivery device comprising the subassembly (200) according to any one of the preceding claims.
